(19) Europäisches Patentamt
European Patent Office
Office européen des brevets

(11) EP 4 397 974 A1

(12) **EUROPEAN PATENT APPLICATION**
published in accordance with Art. 153(4) EPC

(43) Date of publication:
**10.07.2024 Bulletin 2024/28**

(21) Application number: **22864520.6**

(22) Date of filing: **30.08.2022**

(51) International Patent Classification (IPC):
*G01N 33/536* (2006.01)    *C12M 1/00* (2006.01)
*C12Q 1/00* (2006.01)    *G01N 37/00* (2006.01)

(52) Cooperative Patent Classification (CPC):
**C12M 1/00; C12Q 1/00; G01N 33/536; G01N 37/00**

(86) International application number:
**PCT/JP2022/032474**

(87) International publication number:
**WO 2023/032938 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.08.2021 JP 2021140677**

(71) Applicant: **The University of Tokyo**
**Bunkyo-ku, Tokyo 113-8654 (JP)**

(72) Inventors:
• **NOJI, Hiroyuki**
**Tokyo 113-8654 (JP)**
• **TABATA, Kazuhito**
**Tokyo 113-8654 (JP)**
• **YAGINUMA, Hideyuki**
**Tokyo 113-8654 (JP)**

(74) Representative: **Schwarz & Partner Patentanwälte**
**GmbH**
**Patentanwälte**
**Wipplingerstraße 30**
**1010 Wien (AT)**

(54) **DIGITAL ASSAY METHOD, AND DIGITAL ASSAY KIT**

(57)    Provided is a digital assay method for determining the concentration of a substance in a solution, as a method available for making the operation of digital assay simpler and easier, the method comprising the steps of: (1) providing a substrate having a chamber array region in which 10,000 to 10 billion chambers having a volume of 1 attoliter to 100 femtoliters are arranged, wherein the substrate has light permeability and the chambers recess from a surface of the substrate, (2) introducing a solution containing a substance onto the chamber array region of the substrate, (3) covering the chamber array region of the substrate with an adhesive tape comprising a base layer and an adhesive layer, followed by pressure-bonding the adhesive layer of the adhesive tape against the surface of the substrate, thereby discharging excess of the solution in the chamber array region outside the chamber array region and sealing each of the chambers 2 containing the solution liquid-tight to function as reactors, (4) detecting the presence or absence of the substance in each of the reactors based on fluorescence emitted from a fluorescent substance resulting from the presence of the substance, and (5) determining the concentration of the substance in the solution based on the number of reactors in which the presence of the substance is detected.

FIG. 3

## Description

### Technical field

The present disclosure relates to a digital assay method and a digital assay kit for determining the concentration of a substance in a solution, and more specifically, to, e.g., a digital assay method including a step of using a substrate having a chamber array region, in which a large number of chambers having an extremely small volume are arranged, and an adhesive tape to form a large number of reactors having an extremely small volume on the substrate.

### Background art

As a technology for determining the absolute concentration of a substance in a solution, a digital bioassay is known. The measurement principle of the digital bioassay is that if a solution containing a substance in a low concentration is introduced into a large number of small-volume reactors, the substance is not introduced into most of the reactors but introduced into the remaining reactors at a rate of a single molecule for each reactor. For example, if the substance is an enzyme, when a fluorogenic assay substance, which emits fluorescence through an enzymatic reaction, is introduced into a reactor together with the enzyme, fluorescence signals generated from the reactor due to the enzymatic reaction can be binary, "0" and "1". Then, the number of signals "1" is counted to determine the absolute concentration of the enzyme.

Patent Literature 1 discloses, as a technology applicable to digital bioassay, "a method of detecting a microscopic body stored in a plurality of receptacles formed separately from each other, the method comprising the steps of: (1) introducing a solvent into a space between a lower layer part in which the receptacles are formed and an upper layer part facing a surface of the lower layer part in which the receptacles are formed, wherein the solvent contains the microscopic body, (2) introducing gas into the space to form a droplet of the solvent in the receptacles wherein the droplet contains the microscopic body, and (3) detecting the microscopic body present in the droplet optically, electrically, and/or magnetically". This technology has a feature in that gas is used when droplets containing a microscopic body are formed in receptacles to function as reactors.

### Citation List

### Patent Literature

Patent Literature 1: International Publication No. WO2018/181488

### Summary of Invention

### Technical Problem

An object of the present disclosure is primarily to provide a technology available for making the operation of a digital assay simpler and easier.

### Solution to Problem

To attain the above object, the following [1] to [16] are provided in the disclosure.

[1] A digital assay method for determining a concentration of a substance in a solution, comprising the steps of:

(1) providing a substrate having a chamber array region in which 10,000 to 10 billion chambers having a volume of 1 attoliter to 100 femtoliters are arranged, wherein the substrate has light permeability and the chambers recess from a surface of the substrate,
(2) introducing a solution containing a substance onto the chamber array region of the substrate,
(3) covering the chamber array region of the substrate with an adhesive tape comprising a base layer and an adhesive layer, followed by pressure-bonding the adhesive layer of the adhesive tape on the surface of the substrate, thereby discharging excess of the solution in the chamber array region outside the chamber array region and sealing each of the chambers containing the solution liquid-tight to function as reactors,
(4) detecting the presence or absence of the substance in each of the reactors based on fluorescence emitted from a fluorescent substance resulting from the presence of the substance, and
(5) determining the concentration of the substance in the solution based on the number of reactors in which the presence of the substance is detected.

[2] The method according to [1], wherein the adhesive layer of the adhesive tape is formed of a rubber-based adhesive composition and/or a silicone-based adhesive composition.

[3] The method according to [2], wherein the rubber-based adhesive composition is one or more compositions selected from the group consisting of a styrene-isoprene-styrene block copolymer (SIS) adhesive composition, a styrene-butadiene-styrene copolymer (SBS) adhesive composition, a styrene-ethylene/propylene-styrene block copolymer (SEPS) adhesive composition, a styrene-ethylene/butylene-styrene block copolymer (SEBS) adhesive composition, an ethylene-propylene copolymer (EPM) adhesive composition, an isobutylene polymer (polyisobutylene, PIB) adhesive composition, and an isobutylene-isoprene copolymer (butyl rubber, IIR) adhesive composition.

[4] The method according to [3], wherein the rubber-based adhesive composition is one or more compositions selected from the group consisting of a styrene-isoprene-styrene block copolymer (SIS) adhesive composition, a styrene-butadiene-styrene copolymer (SBS) adhesive composition, a styrene-ethylene/propylene-styrene block copolymer (SEPS) adhesive composition, a styrene-ethylene/butylene-styrene block copolymer (SEBS) adhesive composition, and an isobutylene polymer (polyisobutylene, PIB) adhesive composition.

[5] The method according to any one of [1] to [4], wherein the adhesive layer has a thickness of more than 2 $\mu$m to 1000 $\mu$m or less.

[6] The method according to any one of [1] to [5], wherein the surface of the substrate is constituted of a fluororesin film and having hydrophobicity, and the chambers that recess from the surface are discretely formed of a hydrophobic partition wall consisting of the fluororesin film and a hydrophilic bottom surface having no fluororesin film.

[7] The method according to any one of [1] to [5], wherein the surface of the substrate is hydrophilized.

[8] The method according to any one of [1] to [6], wherein the chambers have a diameter of 0.05 to 1000 $\mu$m and a depth of 0.001 to 1000 $\mu$m, and are arranged at intervals of 5 to 10 $\mu$m.

[9] A digital assay kit for determining a concentration of a substance in a solution, comprising

(A) a substrate having a chamber array region in which 10,000 to 10 billion chambers having a volume of 1 attoliter to 100 femtoliters are arranged, wherein the substrate has light permeability and the chambers recess from a surface of the substrate, and
(B) an adhesive tape to be attached to the chamber array region of the substrate and comprising a base layer and an adhesive layer, wherein the adhesive layer of the adhesive tape is formed of a rubber-based adhesive and/or a silicone-based adhesive.

[10] The kit according to [9], wherein the adhesive layer of the adhesive tape is formed of a rubber-based adhesive composition and/or a silicone-based adhesive composition.

[11] The kit according to [10], wherein the rubber-based adhesive composition is one or more compositions selected from the group consisting of a styrene-isoprene-styrene block copolymer (SIS) adhesive composition, a styrene-butadiene-styrene copolymer (SBS) adhesive composition, a styrene-ethylene/propylene-styrene block copolymer (SEPS) adhesive composition, a styrene-ethylene/butylene-styrene block copolymer (SEBS) adhesive composition, an ethylene-propylene copolymer (EPM) adhesive composition, an isobutylene polymer (polyisobutylene, PIB) adhesive composition, and an isobutylene-isoprene copolymer (butyl rubber, IIR) adhesive composition.

[12] The kit according to [11], wherein the rubber-based adhesive composition is one or more compositions selected from the group consisting of a styrene-isoprene-styrene block copolymer (SIS) adhesive composition, a styrene-butadiene-styrene copolymer (SBS) adhesive composition, a styrene-ethylene/propylene-styrene block copolymer (SEPS) adhesive composition, a styrene-ethylene/butylene-styrene block copolymer (SEBS) adhesive composition, and an isobutylene polymer (polyisobutylene, PIB) adhesive composition.

[13] The kit according to any one of [9] to [12], wherein the adhesive layer has a thickness of more than 2 $\mu$m to 1000 $\mu$m or less.

[14] The kit according to any one of [9] to [13], wherein the surface of the substrate is constituted of a fluororesin film and having hydrophobicity, and the chambers that recess from the surface are discretely formed of a hydrophobic partition wall consisting of the fluororesin film and a hydrophilic bottom surface having no fluororesin film.

[15] The kit according to any one of [9] to [13], wherein the surface of the substrate is hydrophilized.

[16] The kit according to any one of [9] to [15], wherein the chambers have a diameter of 0.05 to 1000 $\mu$m, a depth of 0.001 to 1000 $\mu$m and are arranged at intervals of 5 to 10 $\mu$m.

Advantageous Effect of Invention

In the present disclosure, a technology available for making the operation of a digital assay simpler and easier is provided.

Brief Description of Drawings

[Figure 1] The figure is a view illustrating a chamber array chip.
[Figure 2] The figure is a view illustrating the chamber array chip.
[Figure 3] The figure is a view illustrating the procedure of the digital assay method.
[Figure 4] The figure shows the results of a test for detecting a single molecule of $\beta$-galactosidase ($\beta$gal) when adhesive tapes having adhesive layers formed of rubber-, silicone- and acryl- and urethane-based adhesive compositions were used.
[Figure 5] The figure shows the results of a test for detecting a single molecule of $\beta$-galactosidase ($\beta$gal)

when adhesive tapes having adhesive layers formed of rubber-based adhesive compositions were used.

Description of Embodiments

Now, preferred embodiments for carrying out the invention disclosed herein will be described by referring to the accompanying drawings. Note that, the following embodiments are representative examples of the invention of the disclosure but should not be construed as limiting the range of the invention disclosed herein.

The digital assay method according to the disclosure is a method for determining the concentration of a substance in a solution (hereinafter referred to as a "target substance") including the following steps.

(1) Substrate providing step
(2) Solution introduction step
(3) Sealing step
(4) Detection step
(5) Concentration determination step

1. Substrate providing step

In this step, a substrate having a chamber array region in which 10,000 to 10 billion chambers having a volume of 1 attoliter to 100 femtoliters are arranged (hereinafter referred to as a "chamber array chip") is provided.

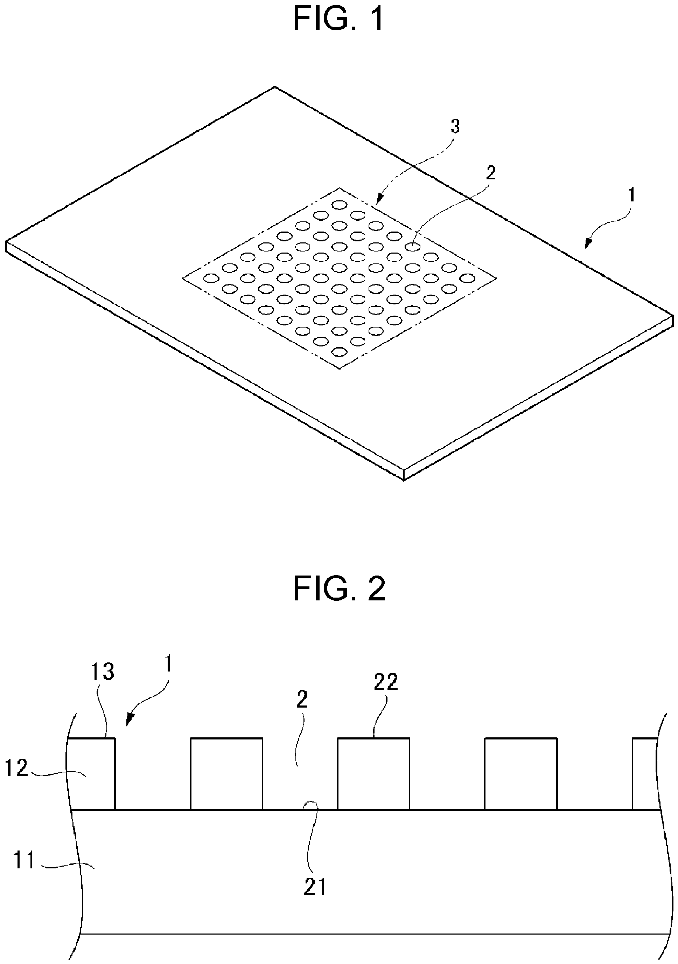

A chamber array chip is shown in Figure 1 and Figure 2.

A chamber array chip 1 has a chamber array region 3 in which a large number of chambers 2 are arranged.

The size and shape of the chamber array chip 1 are not particularly limited. As an example, a rectangular shape of 32 mm in width, 24 mm in depth and 2 mm in thickness is mentioned. The size of the chamber array chip 1 may be 1 to 100 mm in width, 1 to 100 mm in depth and 0.1 to 10 mm in thickness. Examples of the shape of the chamber array chip 1 may include rectangle, square, circle and oval.

The size of the chamber array region 3 is not limited as long as it is smaller than that of the chamber array chip 1 and the shape thereof is not particularly limited. In the above examples, a rectangle having a width of 20 mm and a depth of 20 mm is used. The size of the chamber array region 3 may be 0.1 to 30 mm in width and 0.1 to 30 mm in depth. Examples of the shape of the chamber array region 3 may include a rectangle, square, circle and oval.

The size and shape of chamber 2 are not particularly limited but, for example, a cylindrical or approximately cylindrical shape having a diameter (d) of 4 $\mu$m and a depth (h) of 3 $\mu$m is preferable. The size of the chamber 2 may be 0.05 to 1000 $\mu$m in diameter (d) and 0.001 to 1000 $\mu$m in depth (h). The size and shape of chamber 2 is preferably a cylindrical or prismatic shape in view of moldability. The volume of the chamber 2 is 1 attoliter to 50 nanoliters and preferably 10 femtoliters to 100 femtoliters. The number of chambers 2 to be arranged and the intervals (pitches) thereof in the chamber array region 3 can be appropriately set. The number of chambers to be arranged is, for example, 10,000 to 10 billion and preferably 100,000 to 10 million. The interval (pitch) between the chambers 2 is, for example, 0.05 to 30 $\mu$m and preferably 5 to 10 $\mu$m.

The chamber array chip 1 can be formed by a method known in the technical field such as wet etching or dry etching of a glass substrate or nanoimprint, injection molding and cutting work of a plastic substrate.

The chamber array chip 1 can be obtained by forming patterns of chambers 2 on a substrate 11 preferably by a photolithography method commonly used. More specifically, for example, a fluororesin film 12 is formed on the substrate 11 made of glass by spin coating and baking, and further a photoresist film (not shown in the figure) is formed on the fluororesin film 12. After the photoresist film is irradiated with UV ray through a mask having shapes of chambers 2 patterned there, the substrate 11 is soaked in a resist developer to remove a UV-irradiated portion of the photoresist film. The fluororesin film 12 is removed by dry etching from the portion from which the resist is removed to expose the surface of substrate 11 serving as a bottom surface 21 of the chambers 2. Finally, the substrate 11 is washed to completely remove the resist film. Through the above steps, a large number of chambers 2 constituting of the remaining fluororesin film 12 as the partition wall 22 and the surface of the substrate 11 as the bottom surface 21 can be formed on the substrate 11. The uppermost surface 13 of the chamber array chip 1 obtained is constituted of the remaining fluororesin film 12 as the partition wall 22 and thus hydrophobic. Chambers 2 are formed to recess from the uppermost surface 13 and the bottom surface 21 of the chambers 2 is constituted of the surface of the substrate 11 made of glass and thus hydrophilic.

The uppermost surface 13 of the chamber array chip 1 is preferably hydrophilized by applying a hydrophilic treatment to the fluororesin film 12 constituting the uppermost surface 13. The hydrophilic treatment can be carried out, for example, by coating the fluororesin film 12 with a hydrophilic polymer such as polyvinylpyrrolidone and polyvinyl alcohol, or modifying a functional group of the fluororesin film 12 to be hydrophilic.

Also, the chamber array chip 1 having the hydrophilized uppermost surface 13 can be obtained by using a hydrophilic polymer film in place of a fluororesin film in pattern formation of the chambers 2.

The material for substrate 11 is specified as a light permeable material such as glass or plastic (e.g., PP, PC, PS, COC, COP, PDMS, PMMA, PET, PVC). As the material for the substrate 11, a material yielding less autofluorescence and having fewer optical errors due to low wavelength dispersion is preferably selected.

As the fluororesin for forming the fluororesin film 12, commercially available fluororesin such as CYTOP (registered trademark), TEFLON (registered trademark) AF2400, and TEFLON (registered trademark) AF1600, can be used. CYTOP (registered trademark) is preferable because it is easily microfabricated.

2. Solution introduction step

In this step, a solution containing a target substance is introduced onto the chamber array region 3 of the chamber array chip 1.

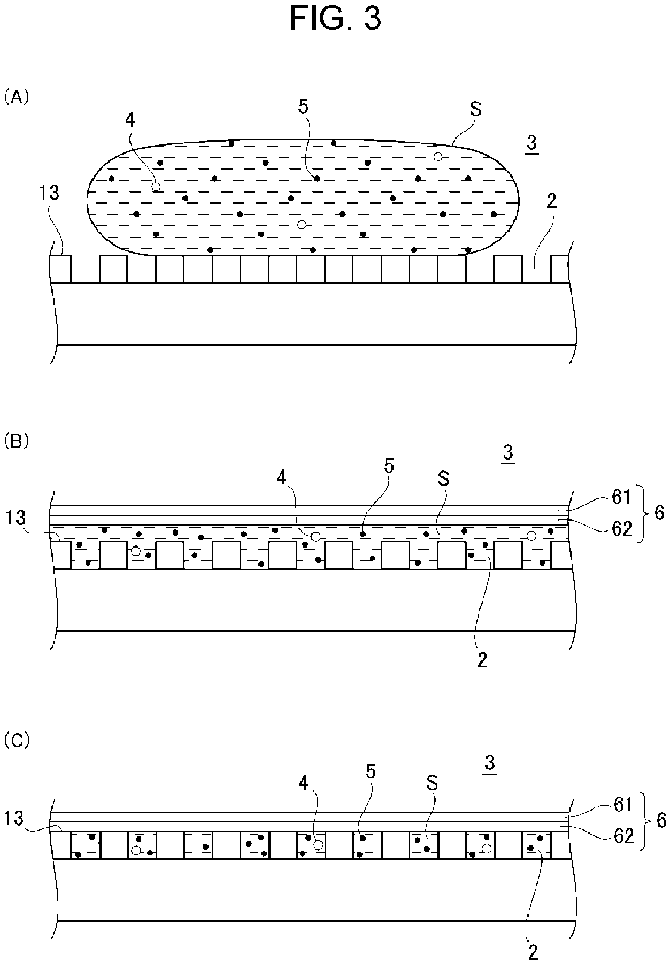

This step will be explained referring to Figure 3.

Solution S containing a target substance 4 is introduced onto the chamber array region 3 of the chamber array chip 1 (see, Figure 3 (A)). Solution S containing a target substance 4 (hereinafter referred to as "sample solution S") may contain a probe 5 generating a fluorescent substance due to the presence of target substance 4. Sample solution S may be introduced, for example, by adding sample solution S dropwise onto the chamber array region 3.

When the uppermost surface 13 of the chamber array chip 1 is hydrophilic, sample solution S added dropwise can be introduced into the interior of each chamber 2.

In contrast, when the uppermost surface 13 of the chamber array chip 1 is hydrophobic, sample solution S added dropwise often remains on the hydrophobic uppermost surface 13 to form droplets, as shown in Figure 3 (A). In such a case, there is no need for sample solution S to completely enter the interior of the chambers 2 in this solution introduction step.

[Target substance]

The target substance 4 is not limited as long as it has a size sufficient to enter the interior of the chambers 2 and may be, e.g., a nucleic acid, a protein, a sugar, a lipid, an amino acid, a peptide, a chemical substance or a complex of these.

The target substance 4 may be a substance serving as a marker for various diseases. In this case, the target substance 4 is particularly, e.g., a virus, a bacterium or a cell.

The marker for a disease may be a substance contained in a biological sample. Examples of the biological sample may include blood, plasma, serum, urea, tear fluid, saliva, and a nasal swab or throat swab.

The probe 5 may be any substance as long as it generates a fluorescent substance due to the presence of the target substance 4, and may be a cell (reporter cell). As the reporter cell, a cell strain modified so as to express a fluorescence/luminescent protein such as GFP or luciferase by a transcription promoter activated in the presence of a protein such as an antibody or a growth factor, and *Escherichia coli* modified so as to express a fluorescence/luminescence protein in the presence of a predetermined chemical substance (e.g., a toxin such as arsenic ion, a metabolite or sugar such as mevalonic acid, fucose and vanillin) are publicly known.

The nucleic acid may be DNA or RNA. The DNA and RNA may be derived from an organism or artificially synthesized and may be cyclic or straight. Examples of the probe 5 that can be used in combination with a nucleic acid include a fluorescently labeled oligo DNA nucleotide and RNA aptamer.

The protein may be, e.g., an enzyme, an antibody, a cell surface protein, proteins derived from other cells, a virus-derived protein, or an artificial protein. Examples of the probe 5 that can be used in combination with a protein include a fluorescently-labeled antibody, reporter cell lineage and RNA aptamer. The enzyme may be an enzyme present on the surface of particularly a virus. Examples of the probe 5 that can be used in combination with an enzyme include fluorogenic assay substrate prepared by binding a substrate to be cleaved with an enzyme and a fluorescent substance. More specifically, when the target substance 4 is an influenza virus, FDG (Fluorescein-D-Galactopyranoside), which is cleaved by reacting with neuraminidase present on the surface of the influenza virus to release a fluorescent substance, can be used as the fluorogenic assay substrate 5.

The sugar may be a natural or artificial sugar chain or monosaccharide. Examples of the probe 5 to be used in combination with a sugar include a fluorescently labeled antibody, lectin, a reporter cell lineage and an RNA aptamer.

The lipid may be an exosome, a liposome, a micelle or a lipid molecule. Examples of the probe 5 to be used in combination with a lipid include a fluorescently labeled antibody, a reporter cell lineage and an RNA aptamer.

The amino acid and peptide may have a structure consisting of a single amino acid or a plurality of amino acids (generally, 50 or less) connected. Examples of the probe 5 to be used in combination with an amino acid and a peptide include a fluorescently labeled antibody, a reporter cell lineage and an RNA aptamer.

Target substance 4 may be held by a carrier. As the carrier, microbeads are commonly used. The term "microbeads" is used synonymously with "particles" and is a commonly known technique in the field. The shape of the microbeads is not particularly limited but usually a sphere. Examples of the material for the microbeads include, but are not particularly limited to, glass, silica gel, polystyrene, polypropylene, membrane and a magnetic material. More specifically, examples of the material include cellulose, a cellulose derivative, an acrylic resin, glass, silica gel, polystyrene, gelatin, polyvinylpyrrolidone, a copolymer of vinyl and acrylamide, polystyrene crosslinked with, e.g., divinylbenzene, polyacrylamide, latex gel, polystyrene dextran, rubber, silicon, plastic, nitrocellulose, cellulose, natural sponge, silica gel, glass, metal plastic, cellulose, cross-linked dextran (Sephadex (trademark)) and agarose gel (Sepharose (trademark)). The beads may be porous.

The fluorescent substance may be any fluorescent substance as long as it is known in the technical field. Examples of the fluorescent substance include 4-methylumbelliferone; 6-carboxy-4',5'-dichloro-2',7'-dimethoxyfluorescein succinimidyl ester; 5-(and-6)-carboxyeosin; 5-carboxyfluorescein; 6-carboxyfluorescein; 5-(and 6-)-carboxyfluorescein; 5-carboxyfluorescein-bis-(5-carboxymethoxy-2-nitrobenzyl) ether, -alanine-carboxamide or succinimidyl ester; 5-carboxyfluorescein succinimidyl ester; 6-carboxyfluorescein succinimidyl ester; 5-(and-6)-carboxyfluorescein succinimidyl ester; 5-(4,6-dichlorotriazinyl)aminofluorescein; 2',7'-difluorofluorescein; eosin-5-isothiocyanate; erythrosine-5-isothiocyanate; 6-(fluorescein-5-carboxamido)hexanoic acid or succinimidyl ester; 6-(fluorescein-5-(and-6)-carboxamido)hexanoic acid or succinimidyl ester; fluorescein-5-EX succinimidyl ester; fluorescein-5-isothiocyanate; fluorescein-6-isothiocyanate; Oregon Green(registered trademark) 488 carboxylic acid or succinimidyl ester; Oregon Green488 isothiocyanate; Oregon Green488-X succinimidyl ester; Oregon Green500 carboxylic acid; Oregon Green500 carboxylic acid, succinimidyl ester or triethylammonium salt; Oregon Green514 carboxylic acid; Oregon Green514 carboxylic acid or succinimidyl ester; Rhodamine Green(registered trademark) carboxylic acid, succinimidyl ester or hydrochloride; Rhodamine Green carboxylic acid, trifluoroacetamide or succinimidyl ester; Rhodamine Green-X succinimidyl ester or hydrochloride; Rhodol Green(registered trademark) carboxylic acid, N,O-bis-(trifluoroacetyl) or succinimidyl ester; bis-(4-)carboxypiperidinyl) sulfone rhodamine or di(succinimidyl ester); 5- (and -6)-carboxynaphthofluorescein, 5-(and -6)-carboxynaphthofluorescein succinimidyl ester; 5-carboxyrhodamine 6G hydrochloride; 6-carboxyrhodamine 6G hydrochloride; 5-carboxyrhodamine 6G succinimidyl ester; 6-carboxyrhodamine 6G succinimidyl ester; 5-(and-6)-carboxyrhodamine 6G succinimidyl ester; 5-carboxy-2',4',5',7'-tetrabromosulfone fluorescein succinimidyl ester or bis-(diisopropylethylammonium) salt; 5-carboxytetramethylrhodamine; 6-carboxytetramethylrhodamine; 5-(and-6)-carboxytetramethylrhodamine; 5-carboxytetramethylrhodamine succinimidyl ester; 6-carboxytetramethylrhodamine succinimidyl ester; 5-(and-6)-carboxytetramethylrhodamine succinimidyl ester; 6-carboxy-X-rhodamine; 5-carboxy-X-rhodamine succinimidyl ester; 6-carboxy-X-rhodamine succinimidyl ester; 5-(and-6)-carboxy-X-rhodamine succinimidyl ester; 5-carboxy-X-rhodamine triethylammonium salt; Lissamine(trademark) rhodamine B sulfonyl chloride; malachite green isothiocyanate; NANOGOLD (registered trademark) mono(sulfosuccinimidyl ester); QSY(registered trademark) 21 carboxylic acid or succinimidyl ester; QSY7 carboxylic acid or succinimidyl ester; Rhodamine Red(trademark)-X succinimidyl ester; 6-(tetramethylrhodamine-5-(and-6)-carboxamido)hexanoic acid succinimidyl ester; tetramethylrhodamine-5-isothiocyanate; tetramethylrhodamine-6-isothiocyanate; tetramethylrhodamine-5-(and-6)-isothiocyanate; Texas Red(registered trademark) sulfonyl; Texas Red sulfonyl chloride; Texas Red-X STP ester or sodium salt; Texas Red-X succinimidyl ester; and X-rhodamine-5-(and-6)-isothiocyanate etc., and combinations of these.

[Solution]

Target substance 4 is dissolved in water or a buffer solution.

Examples of the buffer solution that can be used include, but are not particularly limited to, MOPS (3-(N-morpholino)propanesulfonic acid), MES (2-Morpholinoethanesulfonic acid), ADA (N-(2-Acetamido)iminodiacetic acid), PIPES (Piperazine-1,4-bis (2-ethanesulfonic acid)), ACES (N-(2-Acetamido)-2-aminoethanesulfonic acid), BES(N,N-Bis (2-hydroxyethyl)-2-aminoethanesulfonic acid), TES(N-Tris (hydroxymethyl)methyl-2-aminoethanesulfonic acid), HEPES (4-(2-hydroxyethyl)-1-piperazineethanesulfonic acid), Tris (Tris (hydroxymethyl)aminomethane) and DEA (Diethanolamine).

3. Sealing step

In this step, the chamber array region 3 of the chamber array chip 1 is covered with an adhesive tape 6 and then the adhesive tape 6 is pressure-bonded on the uppermost surface 13 of the chamber array chip 1 to discharge excess of sample solution S on the chamber array region 3 outside the chamber array region 3 and sealing each of the chambers 2 containing sample solution S liquid-tight to function as reactors.

This step will be explained referring to Figure 3.

First, the chamber array region 3 having sample solution S added dropwise thereon is covered with the adhesive tape 6 (see, Figure 3 (B)). The adhesive tape 6 comprises the base layer 61 and the adhesive layer 62. At this coverage time, care must be taken such that the adhesive layer 62 of the adhesive tape 6 is not allowed to be in contact with the uppermost surface 13 of the chamber array chip 1. Sample solution S spreads, as if it is squashed by the adhesive tape 6, in the space formed between the adhesive layer 62 of the adhesive tape 6 and the uppermost surface 13 of the chamber array chip 1 (in the direction from the left to right in the figure) and part of sample solution S is introduced into the interior of the chambers 2 and the interior of the chambers 2 is filled with sample solution S. Excessive sample solution S which fails to enter the interior of the chambers 2 is partly pushed out of the chamber array region 3.

In this case, the introduction of sample solution S into the interior of the chambers 2 is promoted by cooling the chamber array chip 1. The temperature for cooling is satisfactory if it is lower than room temperature, for example, 0 to 10°C, and preferably 2 to 5°C. The chamber array chip 1 can be cooled, for example, by placing the chamber array chip 1 on an aluminum block placed on ice.

To promote the introduction of sample solution S into the interior of the chambers 2, the chamber array chip 1 may be vibrated or air may be vacuumed from the chambers under negative pressure. Alternatively, the introduction of sample solution S into the interior of the chambers 2 can be promoted by adding a surfactant to sample solution S. Note that, these treatments for promoting the introduction of sample solution S can be carried out in the solution introduction step.

Next, the adhesive layer 62 of the adhesive tape 6 is pressure-bonded on the uppermost surface 13 of the chamber array chip 1 (see, Figure 3 (C)). By the operation, excessive sample solution S not introduced into the chambers 2 can be completely discharged outside the chamber array region 3, at the same time, the chambers 2 containing sample solution S is sealed liquid-tight to function as reactors. The adhesive tape 6 can be pressure-bonded on the chamber array chip 1 placed on a stable flat workbench by moving a roller back and forth while applying an appropriate pressing force to the adhesive tape 6.

When the concentration of the target substance 4 in sample solution S is low, each of the chambers 2 either has a single molecule of the target substance 4 introduced therein or does not have it.

The concentration of target substance 4 to be measured usually falls within the range of 1 zM to 1 mM.

In contrast, if the probe 5 is contained in sample solution S, the probe 5 is preferably contained in sample solution S at a sufficiently high concentration compared to the concentration of the target substance 4 such that 1 molecule or 2 molecules or more of the probe 5 are introduced into all chambers 2.

[Adhesive tape]

The adhesive tape 6 used herein preferably has light permeability. However, light permeability is not essential depending on the structure of the optical detection system used in the detection step. More specifically, the adhesive tape 6 may not be light-permeable in the case where irradiation of excitation light and detection of fluorescence are both carried out upwardly from the lower side of the chamber array chip 1 or in the case where the probe 5 which can emit light without irradiation of excitation light is used and detection is carried out upwardly from the lower side of the chamber array chip 1.

The adhesive tape 6 can be obtained by forming the adhesive layer 62 by applying a solution of an adhesive composition to a base layer 61 and evaporating a solvent. Dissolution of the adhesive composition in a solvent, application of the solution of the adhesive composition to the base layer 61, and drying treatment may be carried out by a method commonly known in the technical field. The solvent (for example, toluene) can be appropriately selected.

The thickness of the base layer is not particularly limited and, for example, 1 to 100 $\mu$m, preferably 2 to 50 $\mu$m, and more preferably 4 to 25 $\mu$m.

The thickness of the adhesive layer is, for example, more than 2 $\mu$m and 1000 $\mu$m or less, preferably 5 to 50 $\mu$m, and more preferably 5 to 20 $\mu$m. If the thickness of 2 $\mu$m or less, unevenness of the uppermost surface 13 of the chamber array chip 1 due to smear and the like, cannot be absorbed by the elasticity of the adhesive layer 62, with the result that the success rate of assay significantly decreases. If the thickness exceeds 1000 $\mu$m, it becomes difficult to form the adhesive layer 62 having a uniform thickness. In addition to this, the adhesive layer is often nonuniformly compressed when it is pressure-bonded, leading to local incomplete sealing of the chambers 2.

[Base layer]

Examples of the light permeable material that can be used for the base layer 61 include a film consisting of polyester, PP, polyimide, PET, cellophane, PVC, polyolefin or the like. Examples of the material not light permeable that can be used include paper, non-woven fabric, foam such as urethane foam, and metal foil.

[Adhesive layer]

The adhesive composition for forming the adhesive layer 62 is not particularly limited and can be appropriately selected from compositions commonly used in the technical field, such as rubber-based, silicone-based, acryl-base and urethane-based compositions. A rubber-based adhesive composition or silicone-based adhesive composition is preferable and a rubber-based adhesive composition is more preferable.

Examples of the rubber-based adhesive composition include a styrene-isoprene-styrene block copolymer (SIS) adhesive composition, a styrene-butadiene-styrene copolymer (SBS) adhesive composition, a styrene-ethylene/propylene-styrene block copolymer (SEPS) adhesive composition, a styrene-ethylene/butylene-styrene block copolymer (SEBS) adhesive composition, an ethylene-propylene copolymer (EPM) adhesive composition, an isobutylene polymer (polyisobutylene, PIB) adhesive composition, and an isobutylene-isoprene copolymer (butyl rubber, IIR) adhesive composition.

As the rubber-based adhesive composition, an SIS adhesive composition, an SBS adhesive composition, a SEPS adhesive composition, a SEBS adhesive composition and a PIB adhesive composition are preferable.

As the adhesive composition for forming an adhesive layer, the adhesive compositions mentioned above may be used singly or two or more adhesive compositions mentioned above may be used in combination.

The "SIS adhesive composition" refers to a composition containing SIS as a main polymer. The content rate (mass ratio) of the main polymer in the solid component is at least 30% or more, and preferably 40% or more. The SIS adhesive composition may contain components other than the main polymer, such as a polymer other than the main polymer, a tackifier, a viscosity modifier, a plasticizer, a crosslinking agent, a filler, an anti-aging agent, an antioxidant and an antifoaming agent. The same applies to an SBS adhesive composition, a SEPS adhesive composition, a SEBS adhesive composition, an EPM adhesive composition, a PIB adhesive composition and an IIR adhesive composition.

As the main polymers of SIS, SBS, SEPS, SEBS, EPM, PIB and IIR, commercially available products can be used. Examples of the commercially available products are shown below.

[SIS polymer]

Kraton Corporation, Kraton D SIS, Cat. No.: DX401
ZEON CORPORATION, Quintac, Cat. No.: 3421

[SBS polymer]

Kraton Corporation, Kraton D SBS, Cat. No.: D1155
Asahi Kasei Corporation, styrenic thermoplastic elastomer (SBS) Tufprene, grade A

[SEPS polymer]

Kuraray Co., Ltd., SEPTON, Cat. No.: 2002
Kuraray Co., Ltd., SEPTON, Cat. No.: 2063

[SEBS polymer]

Kuraray Co., Ltd., SEPTON, Cat. No.: 8004

[EPM polymer]

JSR Corporation, JSR EP, grade EP331

[PIB polymer]

ENEOS Holdings Inc. Tetrax, grade 6T

[IIR polymer]

JSR Corporation JSR BUTYL, grade 065
As the tackifier, a tackifier commonly used in the technical field can be appropriately used. Examples of the tackifier include an aliphatic hydrocarbon resin, an aromatic hydrocarbon resin, a terpene resin, and a rosin resin. Examples of the commercially available tackifier are shown below.

ENEOS Holdings Inc., hydrogenated hydrocarbon resin T-REZ H series, Cat. No.: HA085
YASUHARA CHEMICAL Co., Ltd., aromatic terpene resin YS resin TO series, Cat. No.: TO125
YASUHARA CHEMICAL Co., Ltd., terpene resin YS resin PX series, Cat. No.: PX1150N

As the viscosity modifier, plasticizer, crosslinking agent, filler, anti-aging agent, antioxidant and antifoaming agent, products commonly used in the technical field can be appropriately used. Examples of these are shown below.
Examples of the viscosity modifier include paraffin wax; examples of the plasticizer include liquid paraffin, dibutyl phthalate and diisononyl adipate; examples of the crosslinking agent include an isocyanate crosslinking agent; examples of the filler include calcium carbonate, silica, talc and cellulose powder; examples of the anti-aging agent include ditertiary butyl hydroxytoluene and N,N'-diphenyl-p-phenylendiamine; examples of the antioxidant include Irganox1010 (BASF JAPAN LTD.) and ANTAGE HP-400 (Kawaguchi Chemical Industry Co., LTD.); and examples of the antifoaming agent include silicone oils.
The "silicone-based adhesive composition" is an adhesive composition containing a silicone polymer as a main polymer. The content rate (mass ratio) of the silicone polymer in the composition is at least 30% or more, and preferably 40% or more in the solid component. Examples of silicone-based adhesive include addition-reaction type silicone-based adhesive, peroxide cure type silicone-based adhesive, and condensation-type silicone-based adhesive.
Examples of the commercially available silicone-based adhesives are shown below:

[Silicone polymer]

Shin-Etsu Chemical Co., Ltd., Cat. No.: KR-3700
Shin-Etsu Chemical Co., Ltd., Cat. No.: X-40-3237
Shin-Etsu Chemical Co., Ltd., Cat. No.: KR-100
Shin-Etsu Chemical Co., Ltd., Cat. No.: KR-130

The silicone-based adhesive composition may contain components other than a silicone polymer, such as a polymer other than a silicone polymer, a catalyst, a crosslinking agent, an antifoaming agent, a filler, a diluent and a resin modifier.
As the catalyst, a catalyst commonly used in the technical field, such as platinum-based catalyst solution (for example, product name "CAT-PL-50T", Shin-Etsu Chemical Co., Ltd.) can be appropriately used.
As the crosslinking agent, a crosslinking agent commonly used in the technical field such as benzoyl peroxide can be appropriately used.
As the diluent and resin modifier, a diluent and resin modifier commonly used in the technical field can be appropriately used. Examples of the diluent include organic solvents such as toluene. Examples of the resin modifier include a silicone oil, a silicone resin and various silane coupling agents.

4. Detection step

In this step, the presence or absence of the target substance 4 in the chambers 2 is detected based on fluorescence emitted from a fluorescent substance generated due to the presence of the target substance 4.

When the concentration of the target substance 4 in sample solution S is low, each of the chambers 2 either has a single molecule of the target substance 4 introduced therein or does not have it (see, Figure 3(B)). Accordingly, by detecting the fluorescence generated from individual chambers 2 due to the presence of the target substance 4, the fluorescence signals can be binary "0" and "1". In the next concentration determination step, the number of signals determined as "1" is counted to determine the absolute concentration of the target substance 4 in sample solution S.

In the case where the target substance 4 mentioned above is an influenza virus and probe 5 is FDG (Fluorescein-D-Galactopyranoside), fluorescein is released in the chamber 2 having the influenza virus introduced therein by an enzymatic reaction of FDG with neuraminidase of the influenza virus. In contrast, fluorescein is not released in the chamber 2 having no influenza virus introduced therein.

Accordingly, by optically detecting fluorescence of fluorescein emitted from individual chambers 2, binary fluorescent signals 0" and "1" can be obtained.

5. Concentration determination step

Based on the number of chambers (reactors) 2 in which the presence of the target substance 4 is detected, the concentration of the target substance 4 in a solution is determined.

More specifically, provided that the volume of the chamber 2 is V (unit: liter) and the percentage of chambers 2 in which the presence of the target substance 4 is detected relative to the total number of the chambers 2, is expressed by $\lambda$, if $\lambda$ is sufficient small, molar concentration C (unit: M) of the target substance 4 can be obtained by the following formula:

$$C = \lambda / (N \times V)$$

wherein N is the Avogadro's number.

$\lambda$ is theoretically 0 or more and 1 or less but, to determine the concentration of the target substance 4, $\lambda$ is desired to be 0.5 or less and desirably 0.1 or less.

Examples

[Test Example 1: Bioassay using adhesive tapes having adhesive layers formed of rubber-, silicone-, acryl- and urethane-based adhesive compositions]

(1) Production of adhesive tape

(1-1) Preparation of solution of adhesive composition

Solutions of rubber-, silicone-, acryl- and urethane-based adhesive compositions were prepared by the following procedure.

[Rubber-based adhesive composition]

[Styrene-isoprene-styrene block copolymer (SIS) adhesive composition]

(i) Adhesive composition IS03

A SIS polymer (Kraton Corporation, Kraton D SIS, Cat. No.: DX401), a tackifier (ENEOS Holdings Inc., hydrogenated hydrocarbon resin T-REZ H series, Cat. No.: HA085; hereinafter referred to as "HA085") and toluene were mixed in a mass ratio of 1: 1: 13 and stirred while rotating for 72 hours to completely dissolve them to obtain a solution of adhesive composition IS03.

(ii) Adhesive composition IS07

A SIS polymer (ZEON CORPORATION, Quintac, Cat. No.: 3421), a tackifier (YASUHARA CHEMICAL Co., Ltd., aromatic terpene resin YS resin TO series, Cat. No.: TO125) and toluene were mixed in a mass ratio of 1: 1: 13 and stirred while rotating for 72 hours to completely dissolve them to obtain a solution of adhesive composition IS07.

(iii) Adhesive composition IS09

A SIS polymer (ZEON CORPORATION, Quintac, Cat. No.: 3421), a tackifier (YASUHARA CHEMICAL Co., Ltd., terpene resin YS resin PX series, Cat. No.: PX1150N) and toluene were mixed in a mass ratio of 1: 1: 13 and stirred while rotating for 72 hours to completely dissolve them to obtain a solution of adhesive composition IS09.

[Styrene-butadiene-styrene copolymer (SBS) adhesive composition]

(i) Adhesive composition BS02

A SBS polymer (Kraton Corporation, Kraton D SBS, Cat. No.: D1155), a tackifier (HA085) and toluene were mixed in a mass ratio of 1: 1: 13 and stirred while rotating for 72 hours to completely dissolve them to obtain a solution of adhesive composition BS02.

(ii) Adhesive composition BS03

An SBS polymer (Asahi Kasei Corporation, styrenic thermoplastic elastomer (SBS) Tufprene, grade A), a tackifier (HA085) and toluene were mixed in a mass ratio of 1: 1: 13 and stirred while rotating for 72 hours to completely dissolve them to obtain a solution of adhesive composition BS03.

(iii) Adhesive composition BS05

SBS polymer (Asahi Kasei Corporation), a tackifier (HA085) and toluene were mixed in a mass ratio of 1: 1: 13 and stirred while rotating for 72 hours to completely dissolve them to obtain a solution of adhesive composition BS05.

[Styrene-ethylene/propylene-styrene block copolymer (SEPS) adhesive]

(i) Adhesive composition EP01

A SEPS polymer (Kuraray Co., Ltd., SEPTON, Cat. No.: 2002), a tackifier (HA085) and toluene were mixed in a mass ratio of 1: 1: 13 and stirred while rotating for 72 hours to completely dissolve them to obtain a solution of adhesive composition EP01.

(ii) Adhesive composition EP03

A SEPS polymer (Kuraray Co., Ltd., SEPTON, Cat. No.: 2063), a tackifier (HA085) and toluene were mixed in a mass ratio of 1: 1: 13 and stirred while rotating for 72 hours to completely dissolve them to obtain a solution of adhesive composition EP03.

[Styrene-ethylene/butylene-styrene block copolymer (SEBS) adhesive composition]

(i) Adhesive composition EP02

A SEBS polymer (Kuraray Co., Ltd., SEPTON, Cat. No.: 8004), a tackifier (HA085) and toluene were mixed in a mass ratio of 1: 1: 13 and stirred while rotating for 72 hours to completely dissolve them to obtain a solution of adhesive composition EP02.

[Ethylene-propylene copolymer (EPM) adhesive composition]

(i) Adhesive composition EPG01

An ethylene/propylene copolymer (JSR CORPORATION, JSR EP, grade EP331), a tackifier (HA085) and toluene were mixed in a mass ratio of 1: 1: 13 and stirred while rotating for 72 hours to completely dissolve them to obtain a solution of adhesive composition EPG01.

[Isobutylene polymer (polyisobutylene, PIB) adhesive composition]

(i) Adhesive composition IB01

Polyisobutylene (ENEOS Holdings Inc. Tetrax, grade 6T), a tackifier (HA085) and toluene were mixed in a mass ratio of 1: 1: 13 and stirred while rotating for 72 hours to completely dissolve them to obtain a solution of adhesive composition IB01.

[Isobutylene-isoprene copolymer (butyl rubber, IIR) adhesive composition]

(i) Adhesive composition BT01

A butyl rubber (JSR CORPORATION, JSR BUTYL, grade 065), a tackifier (HA085) and toluene were mixed in a mass ratio of 1: 1: 13 and stirred while rotating for 72 hours to completely dissolve them to obtain a solution of adhesive composition BT01.

[Silicone-based adhesive composition]

(i) Adhesive composition SC11

A silicone-based adhesive main agent (Shin-Etsu Chemical Co., Ltd., Cat. No.: KR-3700), a platinum catalyst (Shin-Etsu Chemical Co., Ltd., Cat. No.: CAT-PL-50T) and toluene were mixed in a mass ratio of 100: 0.3: 400 and stirred while rotating for 30 minutes to obtain a solution of an adhesive composition SC11.

(ii) Adhesive composition SC12

A silicone-based adhesive main agent (Shin-Etsu Chemical Co., Ltd., Cat. No.: X-40-3237), a platinum catalyst (Cat. No.: CAT-PL-50T), crosslinking agent KS-3802 (Shin-Etsu Chemical Co., Ltd., Cat. No.: X-92-122) and toluene were mixed in a mass ratio of 100: 1: 0.5: 200, and stirred while rotating for 30 minutes to obtain a solution of an adhesive composition SC12.

(iii) Adhesive composition SC13

A silicone-based adhesive main agent (Shin-Etsu Chemical Co., Ltd., Cat. No.: KR-100), benzoyl peroxide (company: TCI, Cat. No.: B3152) and toluene were mixed in a mass ratio of 100: 2: 400, and stirred while rotating for 30 minutes to obtain a solution of an adhesive composition SC13.

(iv) Adhesive composition SC14

A silicone-based adhesive main agent (Shin-Etsu Chemical Co., Ltd., Cat. No.: KR-130), benzoyl peroxide (company: TCI, Cat. No.: B3152) and toluene were mixed in a mass ratio of 100: 2: 400, and stirred while rotating for 30 minutes to obtain a solution of an adhesive composition SC14.

[Acryl-based adhesive composition]

(i) Adhesive composition AC05

An acryl-based solution resin (NIPPON CARBIDE INDUSTRIES CO., INC., Nisetsu, Cat. No.: PE-121), an isocyanate crosslinking agent (NIPPON CARBIDE INDUSTRIES CO., INC., Cat. No.: CK-101) and toluene were mixed in a mass ratio of 100: 3: 100 and stirred while rotating for 30 minutes to obtain a solution of adhesive composition AC05.

(ii) Adhesive composition AC06

An acryl-based solution resin (NIPPON CARBIDE INDUSTRIES CO., INC., Nisetsu, Cat. No.: KP-1282), an isocyanate crosslinking agent (NIPPON CARBIDE INDUSTRIES CO., INC., Cat. No.: CK-101) and toluene were mixed in a mass ratio of 100: 2: 200 and stirred while rotating for 30 minutes to obtain a solution of adhesive composition AC06.

(iii) Adhesive composition AC07

An acryl-based solution resin (NIPPON CARBIDE INDUSTRIES CO., INC., Nisetsu, Cat. No.: KP-1405), an isocyanate crosslinking agent (NIPPON CARBIDE INDUSTRIES CO., INC., Cat. No.: CK-102) and toluene were mixed in a mass ratio of 100: 1: 200 and stirred while rotating for 30 minutes to obtain a solution of adhesive composition AC07.

(iv) Adhesive composition AC08

An acryl-based solution resin (NIPPON CARBIDE INDUSTRIES CO., INC., Nisetsu, Cat. No.: KP-2369), an isocyanate crosslinking agent (NIPPON CARBIDE INDUSTRIES CO., INC., Cat. No.: CK-131) and toluene were mixed in a mass ratio of 100: 2: 100 and stirred while rotating for 30 minutes to obtain a solution of adhesive composition AC08.

(v) Adhesive composition AC09

An acryl-based solution resin (TOYOCHEM CO., LTD., ORIBAIN, Cat. No.: BPS-5213K), an isocyanate crosslinking agent (NIPPON CARBIDE INDUSTRIES CO., INC., Cat. No.: BHS8515) and toluene were mixed in a mass ratio of 100: 2: 100 and stirred while rotating for 30 minutes to obtain a solution of adhesive composition AC09.

(vi) Adhesive composition AC10

An acryl-based solution resin (TOYOCHEM CO., LTD., ORIBAIN, Cat. No.: BPS-5296), a tolylene diisocyanate crosslinking agent (TOYOCHEM CO., LTD., Cat. No.: BHS-8515) and toluene were mixed in a mass ratio of 100: 2: 100 and stirred while rotating for 30 minutes to obtain a solution of adhesive composition AC10.

(vii) Adhesive composition AC11

An acryl-based solution resin (TOYOCHEM CO., LTD., ORIBAIN, Cat. No.: KP-5160) and toluene were mixed in a mass ratio of 100: 100 and stirred while rotating for 30 minutes to obtain a solution of adhesive composition AC11.

(viii) Adhesive composition AC12

An acryl-based solution resin (TOYOCHEM CO., LTD., ORIBAIN, Cat. No.: BPS-5375), a tolylene diisocyanate crosslinking agent (TOYOCHEM CO., LTD., Cat. No.: BHS-8515) and toluene were mixed in a mass ratio of 100: 1.7: 200 and stirred while rotating for 30 minutes to obtain a solution of adhesive composition AC12.

[Urethane-based adhesive composition]

(i) Adhesive composition UR01

A urethane solution resin (TOYOCHEM CO., LTD., Cyabine, Cat. No.: SH-101) and an isocyanate crosslinking agent (TOYOCHEM CO., LTD., Cat. No.: T-501B) were mixed in a mass ratio 100: 4 and stirred while rotating for 30 minutes to obtain a solution of adhesive composition UR01.

(ii) Adhesive composition UR02

A urethane solution resin (TOYOCHEM CO., LTD., Cyabine, Cat. No.: SH-205), and an isocyanate crosslinking agent (TOYOCHEM CO., LTD., Cat. No.: T-501B) were mixed in a mass ratio of 100: 3 and stirred while rotating for 30 minutes to obtain a solution of adhesive composition UR02.

(1-2) Lamination of adhesive layer on base layer

A polyimide film (Du Pont-Toray Co., Ltd., Cat. No.: 100H-A4) having a thickness of 25 μm was cut into pieces of about 10 cm square and used as the base layers.
The base layers were put on a hot plate and the solutions (about 0.5 mL) of individual adhesive compositions were added dropwise onto the centers, respectively. After the base layers were allowed to stand still at room temperature for about 30 minutes to evaporate a solvent, the temperature of the hot plate was raised to perform heat treatment. The heating temperature and time differ depending on the type of adhesive. A rubber-based adhesive was treated at 140°C for 60 minutes; an acryl-based adhesive and a urethane-based adhesive were treated at 140°C for 10 minutes; and a silicone-based adhesive was treated at 140°C for 10 minutes or 90°C for 5 minutes followed by 200°C for 5 minutes. The adhesive layers of the adhesive tapes thus obtained had a thickness within the range of about 5 to 50 $\mu$m.

(2) Production of chamber array chip

A chamber array chip was produced by the following procedure.

A glass substrate (Matsunami Glass Ind., Ltd. cover glass, 24 mm × 32 mm, thickness 0.17 mm) was spin-coated with a fluororesin (Asahi Glass Co., Ltd., CYTOP, Cat No.: CTL-816AP) to form a fluororesin film. The condition of spin coating was 2000 rpm for 30 seconds.

The glass substrate coated with the fluororesin film was baked at 180°C for one hour to allow the fluororesin film and the glass substrate to tightly adhere. The spin coating and baking treatment were repeated in total 4 times to form a fluororesin film having a thickness of about 3 $\mu$m on the glass substrate.

On the fluororesin film, a photoresist film was formed by spin-coating of a positive-type photoresist (AZ Electronic Materials, AZP4903). The condition of the spin coating was set at 4000 rpm for 60 seconds.

The glass substrate having the photoresist film formed thereon was baked at 110°C for one hour to allow the photoresist film and the fluororesin film to tightly adhere.

Next, chamber-shape patterns were formed by photolithography. The photoresist film was irradiated with UV ray through a mask having chamber-shape patterns. The glass substrate was soaked in a resist developer to remove a UV-irradiated portion of the photoresist film.

The substrate was set in a dry-etching apparatus (SAMCO, IE-10NR). Dry-etching was performed with $O_2$ plasma to remove the fluororesin film of the portion from which a resist was removed to expose the bottom surface made of glass.

The glass substrate was washed with acetone and ethanol to completely remove the photoresist film. In this manner, a chamber array chip having chamber-shape patterns was obtained.

The resultant chamber array chip has a chamber array region (20 × 20 mm) in which about 5.7 million chambers each having 4 $\mu$m in diameter and 3 $\mu$m in height are formed at pitches of 9 $\mu$m.

(3) Digital assay

Using the chamber array chip and adhesive tapes having adhesive layers formed of various adhesive compositions, detection of a single β-galactosidase molecule (βgal) was carried out.

(3-1) Preparation of digital-assay reaction solution

Enzyme, βgal (Merck KGaA, Cat No.: 10745731001) was diluted with an assay buffer (1M MOPS (pH 7.0), 4 mM $CaCl_2$) to form a 10 pM stock solution.

FDG (Thermo Fisher Scientific, Fluorescein-D-Galactopyranoside, Cat No.: F1179) serving as a fluorogenic assay substrate of βgal was dissolved in DMSO (FUJIFILM Wako Pure Chemical Corporation, dimethyl sulfoxide, Cat. no.: 049-07213) to produce a 25 mM stock solution.

Alexa Fluor 647 (Thermo Fisher Scientific, Alexa Fluor 647 C2 Maleimide, Cat No.: A20347) as a fluorescent dye was dissolved in water to produce a 200 $\mu$M stock solution.

A digital-assay reaction solution was prepared by mixing the assay buffer (16.7 $\mu$L), the βgal stock solution (2 $\mu$L), the FDG stock solution (0.8 $\mu$L) and the fluorescent dye stock solution (0.5 $\mu$L) just before use. The final βgal concentration was 1 pM.

(3-2) Introduction of digital-assay reaction solution into chambers

The digital-assay reaction solution was added dropwise in the chamber array region in the chamber array chip. Since the surface of the chamber array is formed of a fluorine film resin and thus hydrophobic, the digital-assay reaction solution added dropwise remains as droplets without spreading.

The chamber array region was covered with an adhesive tape cut into a piece of about 10 × 20 mm. At this time, care must be taken such that the adhesive layer of the adhesive tape is not allowed to be in contact with the surface of the chamber array chip. The droplets of the digital-assay reaction solution are squashed by the adhesive tape and spread.

The chamber array chip of this state is allowed to stand on an aluminum block placed on ice for about one minute. This operation promotes the introduction of the digital-assay reaction solution into the chambers of the chamber array chip.

(3-3) Sealing of chambers

The chamber array chip is put on a stable flat workbench and a roller is moved back and forth about 5 to 10 times while applying a force of about 10 to 20 kg to the adhesive tape, with the result that the adhesive layer of the adhesive tape is pressurized and attached onto the surface of the chamber array chip. By this operation, individual chambers are sealed liquid-tight and constituted as reactors. Also, by this operation, excessive digital-assay reaction solution on the chamber array region is discharged outside the chamber array region.

(3-4) Digital counting

The chamber array chip was set in a fluorescence microscope and observed. The experimental microscope used for observation is principally equipped with a main body (Olympus Corporation Co., Ltd. inverted research microscope IX83), an LED light source unit for excitation (company: EXCELITAS TECHNOLOGIES, Xcite XYLIS), a high sensitivity sCMOS camera (company: ANDOR, Neo), and a 20X objective lens (Olympus Corporation Co., Ltd. UplanSApo 20x/0.75), etc.

Ten minutes after the chambers were sealed, images in the bright field, fluorescence images of fluorescein and Alexa Fluor 647 were obtained.

In the case of fluorescein (excitation peak wavelength: 490 nm, fluorescence peak wavelength: 525 nm), the setting for GFP observation of a microscope was employed and an exposure time was set at 150 milliseconds, whereas in the case of Alexa Fluor 647 (excitation peak wavelength 651 nm, fluorescence peak wavelength 671 nm), the setting for Cy5 observation was employed and an exposure time was set at 50 milliseconds.

Images were converted by free software ImageJ/Fiji (the National Institutes of Health) for image analysis and displayed, and then, the number of bright spots photographed was counted.

In the center of an image, a test circle of 527 $\mu$m in diameter (equivalent to 3/4 of the short side of the rectangle image photographed) was drawn. The number of bright spots within the circular range in about 3070-3080 chambers was counted.

The photographed fluorescein images were observed. Each chamber emitting fluorescent light was visually checked as to whether a bright spot is due to the generation/accumulation of fluorescein inside the chamber or as to whether a bright spot is due to bubbles or autofluorescence emitted from debris. The image of Alexa Fluor 647 photographed was principally used for assisting the determination of whether a spot is a bubble or not.

In this manner, of chambers (about 3070-3080 chambers) in a test circle, the number of chambers having bright spots due to generation/accumulation of fluorescein generated by the $\beta$gal reaction was counted and used as measured values. The measurement was carried out in 2 to 6 test circles per adhesive tape (data was obtained in a single point per test circle).

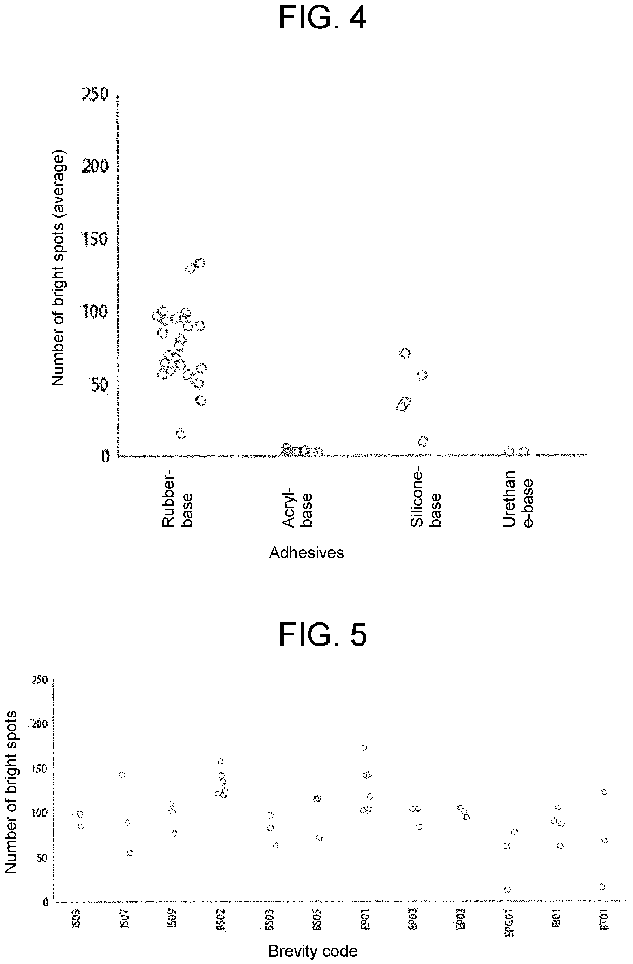

The results are shown in Figure 4.

In test groups using adhesive tapes having adhesive layers formed of rubber-based and silicone-based adhesive compositions (rubber-based adhesive group and silicone-based adhesive group), it was possible to detect a single $\beta$gal molecule. The number of bright spots detected was larger in the rubber-based adhesive group than in the silicone-based adhesive group.

The average number of bright spots in a rubber-based adhesive group fell within the range of 13 to 131. The average number of bright spots in all rubber-based adhesive groups was 74. The average number of bright spots in a silicone-based adhesive group fell within the range of 7.5 to 68. The average number of bright spots in all silicone-based adhesive groups was 39.

In contrast, in test groups using adhesive tapes having adhesive layers formed of acryl- and urethane-based adhesive compositions (acryl-based adhesive group and urethane-based adhesive group), virtually no bright spots were found.

The average number of bright spots in an acryl-based adhesive group fell within the range of 0 to 3. The average number of bright spots in all acryl-based adhesive groups was 0.8. In the urethane-based adhesive group, no bright spots were detected.

This is considered because, in the acryl-based adhesive group and urethane-based adhesive group, chambers containing a digital-assay reaction solution are not completely sealed or leakage of fluorescein generated/accumulated in a reactor due to the $\beta$gal reaction to the adhesive layer of the adhesive tape occurs.

[Test Example 2: Examination of rubber-based adhesive]

The results of Test Example 1 were analyzed again for rubber-based adhesive composition.

The results are shown in Figure 5.

The numbers of bright spots in the SIS group (IS03, IS07, IS09), SBS group (BS02, BS03, BS05), SEPS group (EP01, EP03), SEBS group (EP02) and PIB group (IB01) were all 52 or more.

In contrast, the numbers of bright spots in the EPM group (EPG01) and IIR group (BT01) fell within the range of 10 to 119 and, in some cases, lower than 52.

Among the rubber-based adhesive groups, particularly in the SIS group, SBS group, SEPS group, SEBS group and PIB group, a single $\beta$gal molecule was satisfactorily detected. The number of bright spots was large particularly in the SBS group, SEPS group and SEBS group.

Reference Signs List

    1: Chamber Array Chip
    11: Substrate
    12: Fluororesin film
    13: Uppermost surface
    2: Chamber
    21: Bottom surface
    22: Partition-wall
    3: Chamber array region
    4: Target substance
    5: Probe
    6: Adhesive tape
    61: Base layer

62: Adhesive layer
S: Sample solution

## Claims

1. A digital assay method for determining a concentration of a substance in a solution, comprising the steps of:

    (1) providing a substrate having a chamber array region in which 10,000 to 10 billion chambers having a volume of 1 attoliter to 100 femtoliters are arranged, wherein the substrate has light permeability and the chambers recess from a surface of the substrate,
    (2) introducing a solution containing a substance onto the chamber array region of the substrate,
    (3) covering the chamber array region of the substrate with an adhesive tape comprising a base layer and an adhesive layer, followed by pressure-bonding the adhesive layer of the adhesive tape on the surface of the substrate, thereby discharging excess of the solution in the chamber array region outside the chamber array region and sealing each of the chambers containing the solution liquid-tight to function as reactors,
    (4) detecting the presence or absence of the substance in each of the reactors based on fluorescence emitted from a fluorescent substance resulting from the presence of the substance, and
    (5) determining the concentration of the substance in the solution based on the number of reactors in which the presence of the substance is detected.

2. The method according to claim 1, wherein the adhesive layer of the adhesive tape is formed of a rubber-based adhesive composition and/or a silicone-based adhesive composition.

3. The method according to claim 2, wherein the rubber-based adhesive composition is one or more compositions selected from the group consisting of

    a styrene-isoprene-styrene block copolymer (SIS) adhesive composition,
    a styrene-butadiene-styrene copolymer (SBS) adhesive composition,
    a styrene-ethylene/propylene-styrene block copolymer (SEPS) adhesive composition,
    a styrene-ethylene/butylene-styrene block copolymer (SEBS) adhesive composition,
    an ethylene-propylene copolymer (EPM) adhesive composition,
    an isobutylene polymer (polyisobutylene, PIB) adhesive composition, and
    an isobutylene-isoprene copolymer (butyl rubber, IIR) adhesive composition.

4. The method according to claim 3, wherein the rubber-based adhesive composition is one or more compositions selected from the group consisting of

    a styrene-isoprene-styrene block copolymer (SIS) adhesive composition,
    a styrene-butadiene-styrene copolymer (SBS) adhesive composition,
    a styrene-ethylene/propylene-styrene block copolymer (SEPS) adhesive composition,
    a styrene-ethylene/butylene-styrene block copolymer (SEBS) adhesive composition, and
    an isobutylene polymer (polyisobutylene, PIB) adhesive composition.

5. A digital assay kit for determining a concentration of a substance in a solution, comprising

    (A) a substrate having a chamber array region in which 10,000 to 10 billion chambers having a volume of 1 attoliter to 100 femtoliters are arranged, wherein the substrate has light permeability and the chambers recess from a surface of the substrate, and
    (B) an adhesive tape to be attached to the chamber array region of the substrate and comprising a base layer and an adhesive layer, wherein the adhesive layer of the adhesive tape is formed of a rubber-based adhesive and/or a silicone-based adhesive.

# FIG. 1

# FIG. 2

# FIG. 3

(A)

(B)

(C)

# FIG. 4

# FIG. 5

## INTERNATIONAL SEARCH REPORT

| International application No. |
|---|
| **PCT/JP2022/032474** |

### A. CLASSIFICATION OF SUBJECT MATTER

*G01N 33/536*(2006.01)i; *C12M 1/00*(2006.01)i; *C12Q 1/00*(2006.01)i; *G01N 37/00*(2006.01)i
FI: G01N33/536 D; G01N37/00 102; C12M1/00 A; C12Q1/00 Z

According to International Patent Classification (IPC) or to both national classification and IPC

### B. FIELDS SEARCHED

Minimum documentation searched (classification system followed by classification symbols)

G01N33/536; C12M1/00; C12Q1/00; G01N37/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| Y | JP 2014-170011 A (QUANTERIX CORP.) 18 September 2014 (2014-09-18)<br>see entire text, all drawings, particularly, claims, paragraphs [0096]-[0102], fig. 1, 6, etc. | 1-5 |
| Y | JP 2020-509345 A (ABBOTT LABORATORIES) 26 March 2020 (2020-03-26)<br>see entire text, all drawings, particularly, claims, paragraphs [0051]-[0053], [0065], etc. | 1-5 |
| Y | WO 2002/025289 A1 (I-CARD CORP.) 28 March 2002 (2002-03-28)<br>see entire text, all drawings, particularly, claims, page 24, lines 8-44, fig. 15, etc. | 1-5 |
| Y | JP 2006-090876 A (CSTEC KK) 06 April 2006 (2006-04-06)<br>see entire text, all drawings, particularly, paragraph [0018], etc. | 1-5 |
| Y | JP 2005-065624 A (NITTO DENKO CORP.) 17 March 2005 (2005-03-17)<br>see entire text, all drawings, particularly, paragraphs [0021]-[0023], etc. | 1-5 |
| Y | JP 11-199840 A (KUREHA CHEM. IND. CO., LTD.) 27 July 1999 (1999-07-27)<br>see entire text, all drawings, etc. | 1-5 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 November 2022** | **15 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2022/032474**

### C. DOCUMENTS CONSIDERED TO BE RELEVANT

| Category* | Citation of document, with indication, where appropriate, of the relevant passages | Relevant to claim No. |
|---|---|---|
| P, X | YAGINUMA, Hideyuki and 4 others. A microreactor sealing method using adhesive tape for digital bioassays. Lab Chip. 2022, vol. 22, no. 10, pp. 2001-2010, PMID: 35481587, DOI: 10.1039/d2lc00065b<br>see entire text, all drawings, p. 2002, right column, first paragraph to p. 2003, right column, first paragraph, fig. 1, 2, etc. | 1-5 |

INTERNATIONAL SEARCH REPORT
Information on patent family members

International application No.

**PCT/JP2022/032474**

| Patent document cited in search report | | | Publication date (day/month/year) | Patent family member(s) | | | Publication date (day/month/year) |
|---|---|---|---|---|---|---|---|
| JP | 2014-170011 | A | 18 September 2014 | US | 2011/0212848 | A1 | |
| | | | | see entire text, all drawings, particularly, claims, paragraphs [0116]-[0122], fig. 1, 6, etc. | | | |
| | | | | US | 2012/0277114 | A1 | |
| | | | | US | 2012/0289428 | A1 | |
| | | | | US | 2018/0017552 | A1 | |
| | | | | US | 2019/0302109 | A1 | |
| | | | | US | 2020/0393457 | A1 | |
| | | | | WO | 2011/109364 | A2 | |
| | | | | EP | 2542891 | A1 | |
| | | | | EP | 2995955 | A1 | |
| | | | | EP | 3330709 | A1 | |
| | | | | EP | 3943941 | A1 | |
| | | | | JP | 2013-521499 | A | |
| JP | 2020-509345 | A | 26 March 2020 | US | 2018/0207640 | A1 | |
| | | | | see entire text, all drawings, particularly, claims, paragraphs [0056]-[0058], [0070], etc. | | | |
| | | | | US | 2020/0001300 | A1 | |
| | | | | WO | 2018/136509 | A1 | |
| | | | | EP | 3570980 | A1 | |
| WO | 2002/025289 | A1 | 28 March 2002 | US | 2003/0180191 | A1 | |
| | | | | see entire text, all drawings, particularly, claims, paragraph [0176], fig. 15, etc. | | | |
| | | | | EP | 1333286 | A1 | |
| JP | 2006-090876 | A | 06 April 2006 | (Family: none) | | | |
| JP | 2005-065624 | A | 17 March 2005 | (Family: none) | | | |
| JP | 11-199840 | A | 27 July 1999 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018181488 A